(19) Europäisches Patentamt
European Patent Office
Office européen des brevets

(11) Publication number: **0 177 999**
**B1**

(12) # EUROPEAN PATENT SPECIFICATION

(45) Date of publication of patent specification: **18.01.89**

(51) Int. Cl.$^4$: **C 07 C 2/36,** C 07 C 2/08 // B01J31/24

(21) Application number: **85201437.2**

(22) Date of filing: **10.09.85**

(54) **Ethylene oligomerization process carried out in a diol-based solvent containing monohydric alcohol or water.**

(30) Priority: **09.10.84 US 658949**
**09.10.84 US 659207**

(43) Date of publication of application:
**16.04.86 Bulletin 86/16**

(45) Publication of the grant of the patent:
**18.01.89 Bulletin 89/03**

(84) Designated Contracting States:
**BE DE FR GB IT NL**

(56) References cited:
**DE-A-3 027 782**
**US-A-3 647 915**
**US-A-3 676 523**
**US-A-3 825 615**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Lutz, Eugene Frederick**
**11135 Tupperlake**
**Houston, TX (US)**
Inventor: **Gautier, Pieter Antoine**
**Badhuisweg 3**
**NL-1031 CM Amsterdam (NL)**

(74) Representative: **Aalbers, Onno et al**
**P.O. Box 302**
**NL-2501 CH The Hague (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 177 999 B1

**Description**

The invention relates to a process for the preparation of linear alpha-olefin oligomers of ethylene by reacting ethylene at elevated temperature and pressure in a polar diol-based solvent and in the presence of a catalyst which is a chelate of nickel with a bidentate ligand.

Linear mono-olefins are compounds having established utility in a variety of applications. For example, linear mono-olefins having about 8 to 20 carbon atoms per molecule are known to be particularly useful as intermediates in the production of surfactants, including, for example, olefin sulphonates and alcohol ethoxylates, and of lubricants and plasticizers.

It is known in the prior art to prepare linear mono-olefins by oligomerizing ethylene at elevated temperature and pressure in a polar reaction solvent containing a catalytic nickel complex. Particularly useful as catalysts for this service are the complexes prepared as the reaction product of an olefinic nickel compound and a bidentate ligand. The olefinic nickel compound is suitably a reduced nickel compound or a π-allyl nickel compound. The art expresses preference for use as reaction solvent in such a process of a dihydric alcohol solvent, particularly an aliphatic diol of 2 to 7 carbon atoms per molecule. Illustrative ethylene oligomerization processes employing a nickel complex catalyst and a polar solvent are described in U.S. patent specification 3,676,523, 3,686,351, 3,737,475, 3,644,564, 3,647,914, 3,647,915, 3,825,615, 4,020,121 and 4,260,844.

The present invention centers on the use for one such oligomerization process of a diol-based solvent which comprises a specific combination of both monohydric and dihydric alcohol components, or comprises a limited quantity of water as a reaction promoter. There are disclosures in the above-referenced patent specifications of the use of various monohydric alcohols as solvents for the subject process. Use of monohydric alkanols has, however, been considered to have disadvantage in such a process, when compared to the performance of dihydric alcohols, for instance, from the standpoint of the rate of ethylene oligomerization. A clear preference has existed for the use of process solvents in this service which consist essentially of the dihydric alcohols. Furthermore, oligomerization processes have been practised under essentially anhydrous conditions. Although small amounts of water are commonly introduced into the reaction system together with the catalyst or the solvent make-up, efforts have consistently been made to hold the water content of the diol-based solvent to a level of no more than about 0.2 %w, calculated on diol.

Accordingly, the invention provides a process for the preparation of linear alpha-olefin oligomers of ethylene by reacting ethylene at elevated temperature and pressure in a polar diol-based solvent and in the presence of a catalyst which is a chelate of nickel with a bidentate ligand, characterized in that a diol-based solvent is used which contains a compound of the general formula:

$$ROH \qquad (I)$$

in which R represents a lower alkyl group and that the reaction is carried out under a partial pressure of ethylene of at least 56.2 bar in a solvent mixture which comprises between 40 and 82 per cent by weight of one or more lower aliphatic dihydric alcohols and between 18 and 60 per cent by weight of one or more lower aliphatic monohydric alcohols, said percentages by weight being calculated on the total weight of the one or more lower aliphatic dihydric alcohols and the aliphatic monohydric alcohols in the solvent mixture, or, alternatively, R in the general formula I represents a hydrogen atom and the reaction is carried out in a diol-based solvent which contains in the range of from 0.5 to 4 per cent by weight of water, calculated on diol.

The inventors have discovered that such use of a reaction solvent mixture results in improvement in the rate of ethylene oligomerization and/or modification of the carbon number distribution of the product oligomer mixture and that desirable results in this regard are critically dependent upon the processing conditions stated hereinbefore.

Oligomerization processes in which either the solvent contains proportions of the monohydric and dihydric alcohol components outside of the specified weight ranges or the ethylene partial pressure is less than 56.2 bar do not result in any significant degree of reaction modification with respect to either the rate of oligomer formation or product oligomer carbon number distribution. Several other processing conditions have additionally been observed to contribute to obtaining full advantage of the benefits associated with the use of a dihydric alcohol and monohydric alcohol reaction solvent mixture.

It has also been found that the presence of water in a specified proportion relative to the solvent substantially enhances the rate of the ethylene oligomerization reaction. For example, reaction rates are increased by as much as 30 to 50 per cent, under preferred operation.

Only small amounts of water, i.e., between 0.5 and 4.0 per cent by weight (%w) relative to diol, are sufficient and necessary to achieve the desired rate enhancement. Neither lesser nor greater proportions of water are suitable for this purpose. In the amount specified, water is readily soluble in the diol-based solvent and is compatible with other components of the oligomerization system.

The inventors have, furthermore discovered that the presence of water can be practised to achieve the desired rate enhancement without a concomitant drawback in the distribution in the product of the various ethylene oligomers. In general practice, the presence of the specified small amount of water is found to have a potential drawback from the standpoint of the desirability of the production of oligomers having a

certain carbon number distribution. It has, however, been found that in certain preferred embodiments the invention can be practised, under quantitative limitation on catalyst nickel and ligand components, without significant sacrifice in product oligomer distribution.

The process of this invention is broadly applicable to any ethylene oligomerization process employing a diol reaction solvent and a nickel complex catalyst.

Oligomers are addition products which contain two or more of the monomer units (in this case, two or more ethylene units), but not as many units as the relatively high molecular weight addition products which are referred to as polymers. The present invention is particularly adapted for the production of linear mono-olefinic oligomers of ethylene containing from 2 to about 20 monomer units (i.e., from 4 to about 40 carbon atoms per molecule).

Catalysts suitable for use in the invention are complexes of nickel comprising an atom of nickel chelated with a bidentate chelating ligand. Such catalysts are typically prepared by reacting a suitable bidentate ligand either with an olefinic nickel compound such as bis(cyclooctadiene)nickel (*O*) or a π-allyl nickel compound, or, more preferably, with a simple divalent nickel salt and a reducing agent, e.g., boron hydride, in the presence of ethylene and in a suitable polar organic solvent. Preparation and use of catalysts of the former type are described in U.S. patent specifications 3,644,564, 3,647,914 and 3,647,415. Preparation and use of catalysts of the latter type are described in U.S. patent specifications 3,676,523, 3,686,351, 3,737,475 and 3,825,615.

A notable feature of the each such ethylene oligomerization process catalyzed by a chelate of nickel with a bidentate ligand, is a product mixture in which the several oligomers have a characteristic carbon number distribution. The aforementioned patent specifications, for example, describe a distribution pattern for the individual olefinic oligomers in any given product which is in a geometric form and can be approximated by a single constant, referred to as the "product distribution constant" or "K-factor," according to the mathematical expression:

$$K = \frac{\text{mol of } C_{n+2} \text{ olefin}}{\text{mol of } C_n \text{ olefin}} \; ; \; (\text{for } n = 4, 6, 8 \ldots\ldots).$$

A relatively high K-factor, e.g., on the order of about 0.75 to 0.85, is often desirable to maximize the production of olefins in the carbon number range of about $C_{10}$ to $C_{20}$. It is known that this product distribution constant can be influenced by a number of parameters, including the type of bidentate ligand, the concentration of catalyst in the solvent, the degree of ethylene saturation in the reaction solution, the type of reaction solvent, and the reaction conditions of temperature and pressure.

Preferred bidentate chelating ligands for such catalysts are known to include those having a tertiary organophosphorous moiety with a suitable functional group substituted on a carbon atom attached directly to, or separated by no more than two carbon atoms from, the phosphorous atom of the organophosphorous moiety. Representative ligands of this type are the compounds

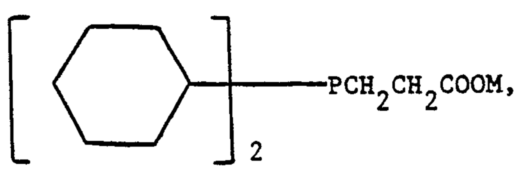

,

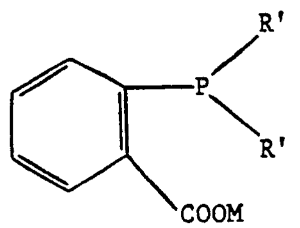

,

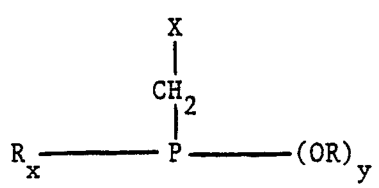

, and

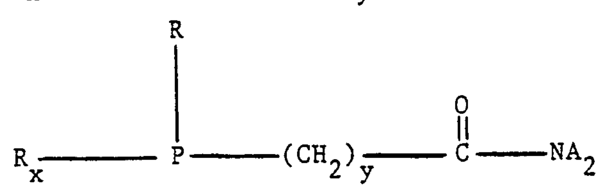

wherein R (independently in each occurrence) represents a monovalent organo group, R' (independently in each occurrence) represents a monovalent hydrocarbyl group, X is carboxymethyl or carboxyethyl, A is hydrogen or a monovalent organo (preferably aromatic) group suitably of up to 10 carbon atoms, M is hydrogen or an alkali metal (preferably sodium or potassium), and x and y (independently) are each either zero, one, or two and the sum of x and y is two, with the proviso that when x is two, the R groups may, together with the phosphorus atom, form a mono or bicyclic heterocyclic phosphine having from 5 to 7 carbon atoms in each ring thereof. Particularly preferred complexes are those described in U.S. patent specification 3,676,523 in which the ligand is an o-dihydrocarbylphosphinobenzoic acid or an alkali metal salt thereof and most preferably o-diphenylphosphinobenzoic acid; in another preferred complex described in U.S. patent specification 3,825,615, the ligand is dicyclohexylphosphinopropionic acid or an alkali metal salt thereof.

Although it is not desired to be bound by any particular theory, it has been suggested that the catalyst molecule undergoes chemical transformation during the course of the oligomerization reaction, possibly involving coordination and/or bonding of ethylene to the nickel moiety. However, the bidentate chelating ligand apparently remains complexed and/or chemically bonded to the nickel moiety during the course of the oligomerization reaction and this complex of the nickel and the chelating ligand is then the effective catalytic species of the oligomerization process. In any event, the bidentate ligand, such as the phosphorus-containing chelating ligand, is considered an essential component of the catalyst and, provided the nickel catalyst contains the required bidentate ligand, the nickel catalyst may be complexed with a variety of additional organic complexing ligands.

As is the case in prior art practice with such nickel chelate catalysts, the molar ratio of nickel to bidentate ligand used in catalyst preparation is preferably at least 1:1, i.e. the nickel is present in equimolar amount or in molar excess. In the preparation of catalyst complexes from a nickel salt, a ligand and a reducing agent, the molar ratio of nickel salt to ligand is suitably in the range from 0.8:1 to 5:1 when R in the general formula I represents a lower alkyl group and from 1:1 to 5:1 when R in the general formula I represents a hydrogen atom, with molar ratios of about 1.0:1 to 3:1 preferred when R in the general formula I represents a lower alkyl group and of 1.5:1 to 3:1 when R in the general formula I represents a hydrogen atom and ratios of about 1:1 especially suitable when R in the general formula I represents a lower alkyl group and of about 2:1 when R in the general formula I represents a hydrogen atom. In these preparations, the reducing agent such as boron hydride is suitably present in equimolar amount or molar excess relative to the nickel salt. For economic reasons, it is preferred that the reducing agent/nickel ratio does not exceed about 15:1. More preferably, this ratio is between about 1:1 and about 10:1, while a ratio of about 1.5:1 is considered particularly preferred. Ratios somewhat below 1:1 are also suitable.

The nickel complex catalysts are suitably preformed by contacting the catalyst precursors in the presence of ethylene in a suitable polar organic diluent or solvent, which is relatively unreactive toward and not reduced by the (boron hydride) reducing agent. In a preferred modification of producing the preferred catalyst complexes as detailed in U.S. patent specifications 3,676,523, 3,686,351, 3,737,475 and 3,825,615, the solvent, nickel salt and ligand are contacted in the presence of ethylene before the addition of reducing agent. It is essential that such catalyst compositions be prepared in the presence of ethylene. The catalysts are suitably prepared at temperatures of about 0° to 50°C, with substantially ambient temperatures, e.g. 10°—30°C preferred. The ethylene pressure and contacting conditions should be sufficient to substantially saturate the catalyst solution. For example, ethylene pressures may be in the range from 1.7 to 104.4 bar or higher. Substantially elevated ethylene pressures, e.g. in the range from 28.6 to 104.4 bar are preferred.

The lower aliphatic dihydric alcohol ("diol") is preferably one selected from the group consisting of $C_2$ to $C_7$ aliphatic diols and mixtures thereof. These suitable diols include, for example, both the vicinal alkanediols such as ethylene glycol, propylene glycol, 2-methyl-1,2-propanediol, 1,2-butanediol and 2,3-butanediol, and the alpha-omega alkanediols such as 1,3-propanediol, 1,4-butanediol, 1,5-pentanediol, 1,6-hexanediol, and 1,7-heptanediol. Alpha-omega alkane diols of 4 to 6 carbon atoms per molecule are preferred solvents, with 1,4-butanediol being particularly preferred. Mixtures of such diols are very suitable for use as the aliphatic dihydric alcohol component of the reaction solvent for purposes of the invention.

While the solvent is glycol-based, it may also suitably contain lesser amounts of other substances which act in some respects as solvents for the ethylene reactant and the catalyst. Such other solvent substances include, for example, impurities commonly present in (essentially anhydrous) commercial diol solvents or in the nickel chelate catalyst preparation and reaction modifiers other than water. As the oligomerization proceeds, the solvent will, of course, contain substantial amounts of ethylene and of oligomerization products and by-products.

The alkanol component is suitably a lower alkanol, preferably one selected from the group consisting of $C_2$ to $C_7$ aliphatic monohydric alcohols and mixtures thereof. The alkanol is preferably either primary or secondary; the presence of tertiary alkanols in the solvent mixture has not been found to be responsible for process rate enhancement, although they are usefully applied to modify the product K-factor. Examples of suitable alkanols include ethyl alcohol, n-propyl alcohol, isopropyl alcohol, n-butyl alcohol, isobutyl alcohol, sec.-butyl alcohol, tert.-butyl alcohol, n-pentyl alcohol, isopentyl alcohol, tert.-pentyl alcohol, amyl alcohol, 2-methyl-2-butanol, 3-methyl-2-butanol, n-hexanol, 4-methyl-1-pentanol and 2-ethyl-1-hexanol. The primary $C_2$ to $C_5$ alkanols are considered most preferred for use in the invention, particularly n-propanol, n-butanol and n-pentanol.

The alkanol solvent components are readily soluble in the suitable diols and the resulting mixture is compatible with other components of the oligomerization process mixture.

In one broader aspect, the invention centers upon modifications in process performance provided by the mixed alkanol and diol solvent system. The lower alkanol and the lower diol in the oligomerization solvent are observed to have a synergistic influence upon process performance (by increasing reaction rate and/or product K-factor) over a critical range of relative properties. Suitable proportions may be conveniently stated in terms of a concentration of alkanol in the solvent which is between 18 and 60 per cent by weight (%w), calculated on total weight of the specified alkanol and diol components present in the solvent, with the diol then making up the remaining 40 to 82 %w. The use of either lesser or greater quantities of alkanol, that is, concentrations below or above this 18 to 60 %w range, do not result in either a significant increase in reaction rate or K-factor, compared to performance with alkanol or diol alone as solvent. As a general rule, solvents comprising between 20 and 45 %w alkanol, relative to total alkanol and diol are preferred from the standpoint of increasing oligomerization reaction rate, while an alkanol concentration of between 20 and 40 %w is more preferred for this objective. Higher concentrations of alkanol, e.g. 35 to 60 %w and particularly 40 to 55 %w, do not generally provide significant increase in reaction rate and are preferred from the standpoint of their performance in increasing product K-factor. Overall, alkanol concentrations in the range from about 20 to 35 %w are considered most preferred for their influence in combining a maximum increase in reaction rate (e.g. 10 to 20% or more) with a moderate increase in K-factor (e.g. 5 to 10%), compared to the performance of a process utilizing the alkanol or the diol alone as a solvent.

The alkanol and diol mixture constitutes the major fraction, preferably the predominant fraction, e.g. 80 %w or more, of the total oligomerization solvent, with the remainder suitably made up of other substances which act in some respects as solvents for the ethylene reactant and the catalyst. A reaction solvent which consists essentially of the specified alkanol and diol components is considered most preferred. As the ethylene reactant is introduced into the process and as the oligomerization reaction proceeds, the solvent will, of course, contain substantial amounts of ethylene and of oligomerization products and by-products.

With exception of the utilization of the mixed alkanol and diol solvent and the criticalities observed for process pressure, the process of the invention may be practiced according to methods and under conditions well known in the prior art, for instance, as described in the above-referenced patent specifications. Very suitably, a mixture of the catalyst and solvent is prepared and charged to an autoclave or similar pressure reactor. The catalyst is typically prepared in a solvent, which is preferably either a diol solvent or an alkanol and diol mixture as specified for purpose of the invention. Ethylene and solvent are introduced and the reaction mixture is maintained with agitation at the desired reaction temperature and pressure.

Process pressure has been found to be a critical aspect of the present invention. Improvements associated with the alkanol and diol solvent mixture are realized only for operation at ethylene partial pressures in excess of 56.2 bar. At lower pressures, the specified alkanol and diol solvent mixture is not responsible for any meaningful effect upon either oligomerization reaction rate or oligomer product distribution. As a rule the degree of benefit realized from practice of the invention increases with increasing ethylene reactant partial pressure. Preference may be expressed for a process in which ethylene partial pressure is in the range from 63.0 to 138.9 bar, while practice in the range from 70.0 to 125 bar is more preferred and practice in the range from 77 to 104 bar is considered most preferred.

Although process temperature is not critical for purposes of the invention, the degree to which the mixed alkanol/diol solvent modifies the oligomerization reaction rate and/or K-factor is dependent upon the reaction temperature. For best overall results, process temperature is preferably at least 80°C, more preferably at least 85°C (particularly between 85°C and 100°C), and most preferably at least 90°C (particularly between 90°C and 105°C).

In another broader aspect, the present invention centers upon an improvement in process performance which is provided by the presence of water in the oligomerization solvent, and the amount of water in the diol-based solvent is a critical aspect of the invention. For good results from the standpoint of reaction rate enhancement, water content of the solvent is in the range from 0.5 to 4.0 per cent by weight, calculated on the weight of the diol. No meaningful rate enhancement is observed for either lesser or greater water content. Water is readily soluble in the diol solvent phase in quantities within the specified range.

It has been the practice in the prior art relative to oligomerization processes of the sort now claimed to utilize an essentially anhydrous diol solvent containing no more than about 0.2 %w water and typically no more than about 0.1 %w water. Water is present up to a level of about 0.1 %w. in the commercially available anhydrous grade of diols heretofore used in oligomer production.

Preferably, the diol-based solvent in the process of the invention contains between about 0.7 and 3.5 %w water, while a water content between about 1.0 and 3.0 %w is more preferred. Considered most preferred is a quantity of water in the range from about 1.0 to 2.5 %w.

Without intending to be bound to one theory or mechanism of operation for the invention, it can be said that the presence of water in the solvent appears to essentially eliminate an induction period which has been characteristic of such oligomerization processes in the prior art. In the absence of water, or in the

presence of only small amounts (e.g., 0.1 %w to 0.2 %w) of water, the maximum rate of oligomerization is not attained until near the end of a batch reaction. Under practice of the invention, on the other hand, the maximum rate occurs early in the course of a batch reaction.

Furthermore, it has been recognized that the use of a large proportion of water in the reaction solvent, in particular, the use of a water-based solvent rather than a glycol-based solvent, leads to a product in which ethylene polymers rather than oligomers predominate. It has now also been found that the K-factor is potentially affected to a significant degree by the presence of small quantities of water in the reaction solvent under practice in accordance with the invention. As a rule, it is considered preferable that the presence of water in the diol-based solvent not induces significant change in the process K-factor, when compared to the results of a process carried out under substantially anhydrous conditions. For instance, a relatively high K-factor, e.g., in the order of about 0.75 to 0.80, is often desirable to maximize the production of olefins in the intermediate carbon number range of about $C_8$ to $C_{20}$ and to minimize the production of higher carbon number polymeric molecules. The presence of water in the solvent phase of the process of the invention is, however, in many cases responsible for a significant decline in K-factor, e.g., a decrease of approximately 5 per cent.

A further important aspect of the invention is the discovery that the reaction rate enhancement associated with the presence of water in the diol-based solvent can be achieved without significant change in the product distribution constant. Specifically, it has been found that rate enhancement benefits can be realized without significant modification of the process K-factor if the process is practised under particular restrictions upon the concentration of nickel and ligand in the diol-based solvent. In this regard, it has been found to be very desirable to limit the ligand concentration to a value up to about 700 ppm and in particular 600 ppm (parts per million by weight, calculated on the weight of solvent) and to limit the molar ratio of nickel to ligand in the reaction solvent to a value greater than about 1.8. More preferably, the ligand concentration is less than about 500 ppm and the molar ratio of nickel to ligand in the solvent is at least about 2.0, while carrying out the process of the invention with a concentration of ligand less than about 400 ppm and with a molar ratio of nickel to ligand of at least about 2.3 is considered most preferred.

Under conventional practice, K-factor is initially relatively high in a batch reaction, but declines as the reaction progresses. In the practice of the invention, K-factor is initially lower than with a dry solvent (e.g., one with no more than about 0.2 %w water) but remains essentially constant throughout a batch process.

With the exception of the presence of water in the diol-based solvent, the process of this invention may be practised according to methods and under conditions well known in the prior art. Very suitably, a mixture of catalyst precursors and diol-based solvent is prepared and charged to an autoclave or similar pressure reactor. The water may suitably be introduced into the diol-based solvent either before, during, or after catalyst preparation and/or addition to the solvent. Ethylene and then sodium borohydride are introduced to complete catalyst formation and the reaction mixture is maintained with agitation at the desired reaction temperature and pressure for oligomerization. Reaction temperature may vary over a wide range, e.g., 25°C to 150°C, but is preferably at least about 70°C, particularly between about 80°C and 110°C. The pressure must be at least sufficient to maintain the reaction mixture substantially in the liquid phase, although excess ethylene will also be present in a vapour phase. As a rule, the total pressure is less significant to the performance of the process than is the partial pressure of ethylene. Under preferred practice, ethylene partial pressure is in the range from about 49.3 to 173 bar, particularly in the range from about 70 to 139 bar. For achieving the greatest benefit from reaction rate enhancement in accordance with the invention, the ethylene partial pressure is most preferably in the range from about 77 to 104 bar.

Contact between ethylene and catalyst in the solvent and under suitable conditions of temperature and pressure is initiated and continued until the desired degree of oligomerization has occurred. The liquid product mixture is then suitably treated according to conventional procedures, typically including separation of an oligomer-rich liquid phase from the diol-based solvent phase, scrubbing of the oligomer-rich phase to remove residual catalyst, de-ethanization of the scrubbed liquid, and further work-up of the de-ethanized product to separate it into various product fractions. The above-referenced patent specifications and also U.S. patent specifications 4,229,607 and 4,284,837 describe suitable procedures for carrying out the several steps of the overall oligomerization process for purposes of the invention, in either a batch or a continuous manner.

The invention is further illustrated by reference to the following Examples (according to the present invention) and Comparative Experiments (not according to the invention).

For each of the Examples 1—11 and Comparative Experiments A—K, a solution of nickel complex catalyst was prepared by mixing solvent, $NiCl_2.6H_2O$, o-diphenylphosphinobenzoic acid, KOH, and sodium borohydride in the presence of ethylene. In each case, addition was made to a 500 ml stirred round bottom flask under nitrogen atmosphere of about 301 g of solvent (either a lower alkanol, i.e. n-propanol or n-butanol, or a lower alkanediol, i.e. 1,4-butanediol (1,4-BD), or an alkanol and alkanediol mixture), about 1.32 g of a solution of $NiCl_2.6H_2O$ in 1,4-BD (0.017 g of Ni per gram of solution); about 8.11 g of a solution of o-diphenylphosphinobenzoic acid in 1,4-BD (0.0128 g of ligand per gram of solution), and about 2.24 g of a solution of KOH in 1,4-BD (0.00905 g of KOH per gram of solution). The resulting clear yellow mixture contained 67—76 ppm nickel, 328—344 ppm ligand, and had molar ratios of nickel to ligand of 1.02—1.18, of sodium borohydride to nickel of 1.17—1.45, and of potassium hydroxide to ligand of 0.98 to 1.06.

The mixture was pressured into a one-litre autoclave, together with 35 g of ethylene, and stirred for

30 min. To the autoclave was then added a solution containing about 0.0227 g of sodium borohydride in 0.400 g of 1,4-BD and 0.400 g of water, followed by a further 2.47 g of butanediol and 87.5 g of ethylene.

The autoclave was rapidly heated to the desired temperature (either 85°C or 95°C) to initiate the oligomerization reaction, and maintained at this temperature for the full reaction period. Ethylene was introduced to maintain desired pressure, e.g. about 92.0 bar or about 63.8 bar or about 50.7 bar. Samples of the reaction mixture were taken after 60 g of ethylene uptake, and in some cases also after 136 g of ethylene uptake, for determination of K-factors.

Examples 1—4

Following the specified procedures, four Examples were carried out at 95°C, utilizing as reaction solvent a mixture of 1,4-butanediol with a lower primary alkanol (n-propanol in Example 1 and n-butanol in Examples 2—4). The oligomerization reaction mixtures contained about 76 %w 1,4-butanediol and 24 %w alkanol, calculated on total weight of diol and alkanol. Each of the examples was conducted under a pressure of about 92.0 bar.

Results in terms of K-factor and reaction rate are reported in Table 1, together with the results of five Comparative Experiments (designated A—E). The Comparative Experiments were carried out under the same conditions and procedures, but utilizing reaction solvents not in accordance with the invention. Reaction rate reported is the maximum during the course of the run and is in terms of the grams of ethylene uptake per litre of catalyst solution per hour.

TABLE 1

| Example No. | Solvent 1) | | Maximum Reaction Rate | K-factor | |
|---|---|---|---|---|---|
| | Alkanol | Diol | | Intermediate 2) | Final 3) |
| 1 | 24 %w n-P | 76 %w 1,4-BD | 479 | 0.80 | 0.76 |
| 2 | 24 %w n-B | 76 %w 1,4-BD | 467 | 0.82 | 0.78 |
| 3 | 24 %w n-B | 76 %w 1,4-BD | 475 | 0.82 | 0.78 |
| 4 | 24 %w n-B | 76 %w 1,4-BD | 469 | 0.84 | 0.80 |
| Comparative Experiment | | | | | |
| A | | 100 %w 1,4-BD | 378 | 0.73 | 0.68 |
| B | | 100 %w 1,4-BD | 360 | 0.71 | 0.67 |
| C | | 100 %w 1,4-BD | 375 | 0.73 | 0.69 |
| D | 14 %w n-B | 86 %w 1,4-BD | 316 | 0.72 | 0.66 |
| E | 100 %w n-P | | 138 | 0.68 | |

In the Tables herein 1), 2) and 3) have the following meaning:

1) n-P represents n-propanol and n-B represents n-butanol.

2) Determined after 60 grams of ethylene uptake.

3) Determined after 136 grams of ethylene uptake.

Examples 5—7

Three Examples were carried out at 95°C under the above-described procedures, utilizing as solvent a mixture of 24 %w n-butanol (n-B) and 76 %w 1,4-butanediol. Process pressure in each case was about 63.8 bar. Results for Examples 5—7 are presented in Table 2, together with the results of four Comparative Experiments (F—I) carried out in a 1,4-butanediol solvent, without the lower alkanol.

TABLE 2

| Example No. | Solvent[1)] Alkanol | Solvent[1)] Diol | Maximum Reaction Rate | K-factor Intermediate[2)] | K-factor Final[3)] |
|---|---|---|---|---|---|
| 5 | 24 %w n-B | 76 %w 1,4-BD | 250 | 0.75 | |
| 6 | 24 %w n-B | 76 %w 1,4-BD | 290 | 0.74 | 0.69 |
| 7 | 24 %w n-B | 76 %w 1,4-BD | 268 | 0.75 | |
| Comparative Experiment | | | | | |
| F | | 100 %w 1,4-BD | 389 | 0.63 | 0.58 |
| G | | 100 %w 1,4-BD | 302 | 0.67 | |
| H | | 100 %w 1,4-BD | 388 | 0.67 | 0.64 |
| I | | 100 %w 1,4-BD | 331 | 0.70 | 0.66 |

Table 2 illustrates practice of the invention under conditions of pressure which result in an increase in K-factor, but not in reaction rate, relative to the Comparative Experiments utilizing only a butanediol solvent.

Examples 8—9

Table 3 presents the results of two Examples and one Comparative Experiment run at a temperature of 85°C and at pressures in the range from 66.1—66.9 bar. The molar ratio of sodium borohydride to nickel used in catalyst preparation for these runs was in the range from 1.50 to 1.58.

TABLE 3

| Example No. | Solvent[1)] Alkanol | Solvent[1)] Diol | Maximum Reaction Rate | K-factor Intermediate[2)] | K-factor Final[3)] |
|---|---|---|---|---|---|
| 8 | 24 %w n-B | 76 %w 1,4-BD | 230 | 0.79 | 0.70 |
| 9 | 48 %w n-B | 52 %w 1,4-BD | 168 | 0.88 | 0.77 |
| Comparative Experiment | | | | | |
| J | 14 %w n-B | 86 %w 1,4-BD | 233 | 0.69 | 0.64 |

Although neither of Examples 8 and 9 according to the invention exhibit increased reaction rate at the temperature (85°C) and pressure (about 66.5 bar) of these runs, both show significant increases in product K-factors over those of Comparative Example J.

Summary of Pressure Effects

Table 4 presents in summary form the results of several Examples and Comparative Experiments to illustrate the critical effect of process pressure upon both maximum reaction rate and K-factor. Except as noted, the reaction rate and K-factor results reported are the averages of several representative runs. The Table illustrates that at a pressure of about 92.0 bar, both the reaction rate and K-factor are increased by practice with a mixed solvent according to the invention, in comparison to practice with a 1,4-butanediol solvent. At a pressure of about 63.8 bar, practice with a mixed solvent under the invention does not increase reaction rate over that attained with the 1,4-butanediol solvent, but does result in a significantly higher K-factor. At the relatively low pressure of about 50.7 bar, not according to the invention, there is no significant difference between the use of a mixed alkanol/diol solvent and the use of a diol solvent alone.

TABLE 4

| Solvent | | Pressure (bar) | Maximum Reaction Rate | K-factor[4)] |
|---|---|---|---|---|
| | 100 %w 1,4-BD | 91.3-93.1 | 371 | 0.72 |
| 24 %w n-B | 76 %w 1,4-BD | 91.3-93.1 | 470 | 0.83 |
| | 100 %w 1,4-BD | 63.8 | 370 | 0.65 |
| 24 %w n-B | 76 %w 1,4-BD | 63.8 | 301 | 0.735 |
| | 100 %w 1,4-BD | 50.0-51.3 | 304[5)] | 0.69 |
| 24 %w n-B | 76 %w 1,4-BD | 50.0-51.3 | 271 | 0.70 |

4) K-factor determined after 60 g ethylene uptake.

5) Result of one experiment.

Examples 10—11

Table 5 presents the results of Examples 10 and 11, together with results of Comparative Experiment K, all run at a temperature of 95°C and at pressures in the range from about 62.0—63.1 bar.

Example 11 illustrates the use of tert.-butyl alcohol (t-B) as the alkanol solvent component. A comparison of results of the three runs shows that practice with a solvent mixture containing tert.-butyl alcohol does not increase reaction rate but does increase K-factor, relative to the use of the 1,4-butanediol alone.

TABLE 5

| Example No. | Solvent[1)] | | Maximum Reaction Rate | K-factor | |
|---|---|---|---|---|---|
| | Alkanol | Diol | | Intermediate[2)] | Final[3)] |
| 10 | 24 %w n-B | 76 %w 1,4-BD | 425 | 0.76 | 0.70 |
| 11 | 24 %w t-B | 76 %w 1,4-BD | 237 | 0.70 | |
| Comparative Experiment | | | | | |
| K | | 100 %w 1,4-BD | 389 | 0.63 | |

Examples 12—14

A series of Examples 12—14 (according to the invention) and Comparative Experiments L and M (not according to the invention) were conducted in a batch mode.

In each case, a solution of nickel complex catalyst in diol-based solvent (95 %w, 1,4-butanediol, 5 %w benzene) was first prepared at ambient temperature by introducing into a stirred stainless steel autoclave reactor: solvent (1,000 g), $NiCl_2 6H_2O$ (500 ml of a 0.3 molar solution in the solvent), KOH (499 g of a 0.1 molar solution in the solvent), and ligand (502 g of a 0.1 molar solution of o-diphenylphosphinobenzoic acid in the solvent. Ethylene was then introduced to a pressure of 35.5 bar, followed by $NaBH_4$ reducing agent (200 g of a 0.8 molar solution in diglyme containing 0.8 g of NaOH), and finally by a further addition of ethylene to bring the pressure to 49.3 bar.

For initiation of the oligomerization reaction, the catalyst solution was heated to the desired temperature (65°C) and pressure in the reactor was adjusted to and maintained at 76.9 bar by further addition of ethylene. After 250 g of ethylene had been taken up by the reaction, a sample of the mixture was withdrawn to monitor K-factor of the intermediate oligomer product. After 500 g of ethylene had been taken up, the liquid mixture in the reactor was drained and allowed to separate into a solvent phase and a final oligomer product phase. K-factors of the intermediate and final products were determined by gas-liquid chromatographic analyses.

Two Comparative Experiments, designated L and M, were carried out to establish the rate of the oligomerization reaction in the diol-based solvent, which contained only about 0.1 %w water. (This 0.1 %w

water was attributed to water impurity in the butanediol, to water in the nickel chloride hydrate and to water resulting from neutralization of KOH.) Examples 12, 13 and 14, all in accordance with the invention, were carried out to illustrate the rate enhancement associated with the presence of greater quantities of water (0.6 %w, 1.1 %w, and 2.1 %w, respectively, calculated on diol-based solvent).

The results (summarized in Table 6) indicate rate enhancement of approximately 30 to 50 per cent for the oligomerization reaction examples in which 0.6 to 2.1 %w water was present in the diol-based solvent, relative to those Comparative Experiments in which minimal water was present in the solvent.

TABLE 6

| Comparative Experiment | Example No. | Solvent, water content, %w | Ethylene Uptake: g per g of ligand per h | Ethylene Uptake: g per l solvent per h | K-factor |
|---|---|---|---|---|---|
| L | | 0.1 | 162 | 130 | 0.74 (1.7 h) |
| | | | | | 0.72 (2.8 h) |
| M | | 0.1 | 151 | 122 | 0.77 (1.7 h) |
| | | | | | 0.76 (2.8 h) |
| | 12 | 0.6 | 214 | 170 | 0.76 (1.5 h) |
| | | | | | 0.74 (2.2 h) |
| | 13[a)] | 1.1 | 233 | 188 | 0.71 (1 h) |
| | 14 | 2.1 | 217 | 169 | 0.71 (1.2 h) |
| | | | | | 0.70 (2.2 h) |

a) In Example 13, water was introduced during catalyst preparation through use of an aqueous $NaBH_4$ solution (3.18 g $NaBH_4$ and 0.40 g NaOH/100 g solution).

Examples 15—21

A further series of Examples 15—21 and Comparative Experiments N—U were carried out in the following manner to illustrate preferred practices of the invention in which reaction rate enhancement is attained without significant change in product K-factor.

For each of Examples 15—18 and Comparative Experiments N—U, a solution of catalyst in 1,4-butanediol solvent was prepared by mixing solvent, $NiCl_26H_2O$, o-diphenylphosphinobenzoic acid, KOH, and sodium borohydride in the proportions indicated in Table 7, with the reducing agent added in the presence of ethylene. In each case, addition was made to a 500 ml stirred round bottom flask under nitrogen atmosphere of: about 301 g of anhydrous 1,4-butanediol (1,4-BD), about 1.32 g of a solution of $NiCl_26H_2O$ in 1,4-BD (0.017 g of Ni per gram of solution); about 8.11 g of a solution of o-diphenylphosphinobenzoic acid in 1,4-BD (0.0128 g of ligand per g of solution) and about 2.24 g of a solution of KOH in 1,4-BD (0.00905 g of KOH per g of solution). The resulting clear yellow mixture was pressured into a one-litre autoclave, together with 35 g of ethylene, and stirred for 30 min. To the autoclave was then added a solution containing about 0.0227 g of sodium borohydride in 0.400 g of 1,4-BD and 0.400 g of water, followed by a further 2.47 g of butanediol and 87.5 g of ethylene. In Examples 19—21 and Comparative Experiments T and U, the same procedures were followed except that the catalyst was formed by adding the $NiCl_2$ and KOH to the solvent, followed by ethylene, $NaBH_4$ and ligand.

Different amounts of water were added to the solvent/catalyst mixture for the several examples. In the comparative experiments, the mixture contained about 0.2 %w water which was introduced with the various components during catalyst preparation.

The autoclave was rapidly heated to 95°C to initiate the oligomerization reaction, and maintained at this temperature for the full reaction period. Ethylene was introduced to maintain a pressure of about 92.7 bar. Samples of the reaction mixture were taken after 60 g of ethylene uptake and after 136 g of ethylene uptake for determination of K-factors.

The results of Examples 15—21 and Comparative Experiments N—U are presented in Table 7, as a function of water content of the solvent. The reaction rate is expressed in terms of the maximum rate of ethylene uptake in g per litre of catalyst/diol solution per h.

TABLE 7

| Comparative Experiment | Example No. | Solvent, water content, %w | maximum reaction rate | K-factor intermediate | K-factor final |
|---|---|---|---|---|---|
| N | | 0.2 | 354 | 0.79 | 0.76 |
| O | | 0.2 | 413 | 0.80 | 0.77 |
| P | | 0.2 | 413 | -- | 0.76 |
| Q | | 0.2 | 435 | -- | 0.76 |
| | 15 | 1.0 | 545 | 0.79 | 0.78 |
| | 16 | 2.0 | 566 | 0.77 | 0.77 |
| | 17 | 2.0 | 558 | 0.77 | 0.77 |
| | 18 | 4.0 | 441 | 0.78 | 0.775 |
| R | | 8.0 | 348 | 0.765 | 0.76 |
| S | | 8.0 | 396 | 0.765 | 0.76 |
| T | | 0.2 | 368 | 0.71 | -- |
| U | | 0.2 | 364 | 0.75 | 0.70 |
| | 19 | 2.1 | 581 | 0.73 | 0.70 |
| | 20 | 2.1 | 576 | 0.71 | 0.69 |
| | 21 | 2.0 | 559 | 0.70 | 0.67 |

**Claims**

1. Process for the preparation of linear alpha-olefin oligomers of ethylene by reacting ethylene at elevated temperature and pressure in a polar diol-based solvent and in the presence of a catalyst which is a chelate of nickel with a bidentate ligand, characterized in that a diol-based solvent is used which contains a compound of the general formula:

$$ROH \qquad (I)$$

in which R represents a lower alkyl group and that the reaction is carried out under a partial pressure of ethylene of at least 56.2 bar in a solvent mixture which comprises between 40 and 82 per cent by weight of one or more lower aliphatic dihydric alcohols and between 18 and 60 per cent by weight of one or more lower aliphatic monohydric alcohols, said percentages by weight being calculated on the total weight of the one or more lower aliphatic dihydric alcohols and the lower aliphatic monohydric alcohols in the solvent mixture, or, alternatively, R in the general formula I represents a hydrogen atom and the reaction is carried out in a diol-based solvent which contains in the range of from 0.5 to 4 per cent by weight of water, calculated on diol.

2. Process as claimed in claim 1, wherein the lower aliphatic dihydric alcohols have 2 to 7 carbon atoms per molecule.

3. Process as claimed in claim 2, wherein the dihydric alcohols are alpha-omega dihydric alcohols having 4 to 6 carbon atoms per molecule.

4. Process as claimed in claim 3, wherein the dihydric alcohol is 1,4-butanediol.

5. Process as claimed in any one of the preceding claims, wherein the lower aliphatic monohydric alcohols have 2 to 7 carbon atoms per molecule.

6. Process as claimed in any one of the preceding claims, wherein the lower aliphatic monohydric alcohols are primary or secondary alcohols.

7. Process as claimed in any one of the preceding claims, wherein the lower aliphatic monohydric alcohols are selected from the group consisting of n-propanol, n-butanol and n-pentanol.

8. Process as claimed in any one of the preceding claims, wherein the solvent mixture comprises between 20 and 45 per cent by weight of the aliphatic monohydric alcohols and between 55 and 80 per cent by weight of the aliphatic dihydric alcohols.

9. Process as claimed in claim 8, wherein the solvent mixture comprises between 20 and 35 per cent by

weight of the monohydric alcohols and between 65 and 80 per cent by weight of the aliphatic dihydric alcohols.

10. Process as claimed in any one of the preceding claims, in which R represents a lower alkyl group and which is carried out at a temperature in the range of 80°C to 105°C.

11. Process as claimed in any one of the preceding claims, in which R represents a lower alkyl group and which is carried out at a partial pressure of ethylene in the range of from 63.0 to 138.9 bar.

12. Process as claimed in claim 1, in which R represents a hydrogen atom and the diol-based solvent contains in the range of from 0.7 to 3.5 per cent by weight of water, calculated on diol.

13. Process as claimed in claim 12, in which the diol-based solvent contains in the range of from 1.0 to 2.5 per cent by weight of water, calculated on diol.

14. Process as claimed in claim 12 or 13, wherein the diol-based solvent contains less than 700 parts per million by weight of the ligand, calculated on total solvent.

15. Process as claimed in any one of the preceding claims, wherein a molar ratio of nickel to ligand of at least 1 is used.

16. Process as claimed in any one of the preceding claims, wherein the bidentate ligand is an o-dihydrocarbylphosphinobenzoic acid or an alkali metal salt thereof.

17. Process as claimed in claim 16, wherein the bidentate ligand is o-diphenylphosphinobenzoic acid or an alkali metal salt thereof.

**Patentansprüche**

1. Verfahren zur Herstellung von linearen α-Olefin Oligomeren von Ethylen durch Umsetzung von Ethylen bei erhöhter Temperatur und (erhöhtem) Druck in einem polaren Lösungsmittel auf Diol-Basis und in Gegenwart eines Katalysators, der ein Chelat ist von Nickel ist einem zweibindigen Liganden, dadurch gekennzeichnet, daß ein Lösungsmittel auf Diol-Basis verwendet wird, das eine Verbindung der allgemeinen Formel

$$ROH \qquad (I)$$

enthält, in der R eine nieder-Alkylgruppe bedeutet und daß die Reaktion unter einem Ethylen-Partialdruck von mindestens 56,2 bar in einem Lösungsmittelgemisch durchgeführt wird, enthaltend zwischen 40 und 82 Gew.-% eines oder mehrerer niederer aliphatischer zweiwertiger Alkohole und zwischen 18 und 60 Gew.-% eines oder mehrerer niederer aliphatischer einwertiger Alkohole, wobei die Gew.-% berechnet sind bezogen auf das Gesamtgewicht des einen oder der mehreren niederen aliphatischen zweiwertigen Alkohole und der niederen aliphatischen einwertigen Alkohole in dem Lösungsmittelgemisch, oder alternativ R in der allgemeinen Formel (I) ein Wasserstoffatom bedeutet und die Reaktion durchgeführt wird in einem Lösungsmittel auf Diol-Basis, das im Bereich von 0,5 bis 4 Gew.-% Wasser enthält, bezogen auf Diol.

2. Verfahren nach Anspruch 1, wobei die niederen aliphatischen zweiwertigen Alkohole 2 bis 7 Kohlenstoffatome pro Molekül besitzen.

3. Verfahren nach Anspruch 2, wobei die zweiwertigen Alkohole α-ω-zweiwertige-Alkohole mit 4 bis 6 Kohlenstoffatomen im Molekül sind.

4. Verfahren nach Anspruch 3, wobei die zweiwertige Alkohol 1,4-Butandiol ist.

5. Verfahren nach einem der vorangehenden Ansprüche, wobei die niederen aliphatischen einwertigen Alkohole 2 bis 7 Kohlenstoffatome im Molekül enthalten.

6. Verfahren nach einem der vorangehenden Ansprüche, wobei die niederen aliphatischen einwertigen Alkohole primäre oder sekundäre Alkohole sind.

7. Verfahren nach einem der vorangehenden Ansprüche, wobei die niederen aliphatischen einwertigen Alkohole ausgewählt sind aus der Gruppe bestehend aus n-Propanol, n-Butanol und n-Pentanol.

8. Verfahren nach einem der vorangehenden Ansprüche, wobei das Lösungsmittelgemisch zwischen 20 und 45 Gew.-% der aliphatischen einwertigen Alkohole und zwischen 55 und 80 Gew.-% der aliphatischen zweiwertigen Alkohole enthält.

9. Verfahren nach Anspruch 8, wobei das Lösungsmittelgemisch zwischen 20 und 35 Gew.-% einwertiger Alkohole und zwischen 65 und 80 Gew.-% der aliphatischen zweiwertigen Alkohole enthält.

10. Verfahren nach einem der vorangehenden Ansprüche, wobei R eine niedere Alkylgruppe bedeutet und das durchgeführt wird bei einer Temperatur im Bereich von 80 bis 105°C.

11. Verfahren nach einem der vorangehenden Ansprüche, wobei R eine niedere Alkylgruppe bedeutet und das durchgeführt wird bei einem Ethylen-Partialdruck im Bereich von 63,0 bis 138,9 bar.

12. Verfahren nach Anspruch 1, wobei R ein Wasserstoffatom bedeutet und das Lösungsmittel auf Diol-Basis im Bereich von 0,7 bis 3,5 Gew.-% Wasser, bezogen auf das Diol, enthält.

13. Verfahren nach Anspruch 12, bei dem das Lösungsmittel auf Diol-Basis im Bereich von 1,0 bis 2,5 Gew.-% Wasser, bezogen auf das Diol, enthält.

14. Verfahren nach Anspruch 12 oder 13, wobei das Lösungsmittel auf Diol-Basis weniger als 700 Gew.-Teile pro Million Ligand, berechnet auf das gesamte Lösungsmittel enthält.

15. Verfahren nach einem der vorangehenden Ansprüche, wobei ein Molverhältnis von Nickel zu Ligand von mindestens 1 angewandt wird.

16. Verfahren nach einem der vorangehenden Ansprüche, wobei der zweibindige Ligand eine o-Dihydrocarbylphosphinbenzoesäure oder ein Alkalimetallsalz davon ist.

17. Verfahren nach Anspruch 16, wobei der zweibindige Ligand o-Diphenylphosphinbenzoesäure oder ein Alkalisalz davon ist.

**Revendications**

1. Procédé pour la préparation d'oligomères alpha-oléfiniques linéaires de l'éthylène par réaction de l'éthylène à une température et une pression élevées dans un solvant polaire à base de diol et en présence d'un catalyseur qui est un chélate de nickel avec un ligand bidentate, caractérisé en ce que l'on utilise un solvant à base de diol qui contient un composé de formule générale

$$ROH \quad (I)$$

dans laquelle R représente un groupe alkyle inférieur et en ce que l'on effectue la réaction sous une pression partielle de l'éthylène d'au moins 56,2 bars dans un mélange de solvants qui comprend entre 40 et 82 pour-cent en poids d'un ou plusieurs alcools dihydroxylés aliphatiques inférieurs et entre 18 et 60 pour-cent en poids d'un ou plusieurs monoalcools aliphatiques inférieurs, lesdits pourcentages pondéraux étant calculés par rapport au poids total du ou des plusieurs alcools dihydroxylés aliphatiques inférieurs et monoalcools aliphatiques inférieurs dans le mélange de solvants, ou d'une autre manière, R dans la formule générale I représente un atome d'hydrogène et on effectue la réaction dans un solvant à base de diol qui contient dans l'intervalle compris entre 0,5 et 4 pour-cent en poids d'eau, calculés par rapport au diol.

2. Procédé selon la revendication 1, dans lequel les alcools dihydroxylés aliphatiques inférieurs ont de 2 à 7 atomes de carbone par molécule.

3. Procédé selon la revendication 2, dans lequel les alcools dihydroxylés sont des alcools dihydroxylés alpha-oméga ayant de 4 à 6 atomes de carbone par molécule.

4. Procédé selon la revendication 3, dans lequel l'alcool dihydroxylé est le 1,4-butanediol.

5. Procédé selon l'une quelconque des revendications précédentes, dans lequel les monoalcools aliphatiques inférieurs ont de 2 à 7 atomes de carbone par molécule.

6. Procédé selon l'une quelconque des revendications précédentes, dans lequel les monoalcools aliphatiques inférieurs sont des alcools primaires ou secondaires.

7. Procédé selon l'une quelconque des revendications précédentes, dans lequel on choisit les monoalcools aliphatiques inférieurs dans le groupe comprenant le n-propanol, le n-butanol et le n-pentanol.

8. Procédé selon l'une quelconque des revendications précédentes, dans lequel le mélange de solvants comprend entre 20 et 45 pour-cent en poids des monoalcools aliphatiques et entre 55 et 80 pour-cent en poids des alcools dihydroxylés aliphatiques.

9. Procédé selon la revendication 8, dans lequel le mélange de solvants comprend entre 20 et 35 pour-cent en poids de monoalcools et entre 65 et 80 pour-cent en poids des alcools dihydroxylés aliphatiques.

10. Procédé selon l'une quelconques des revendications précédentes, dans lequel R représente un groupe alkyle inférieur et que l'on effectue à une température comprise dans l'intervalle allant de 80 à 105°C.

11. Procédé selon l'une quelconque des revendications précédentes, dans lequel R représente un groupe alkyle inférieur et que l'on effectue sous une pression partielle d'éthylène comprise dans l'intervalle allant de 63,0 à 138,9 bars.

12. Procédé selon la revendication 1, dans lequel R représente un atome d'hydrogène et le solvant à base de diol contient dans l'intervalle allant de 0,7 à 3,5 pour-cent en poids d'eau, calculés par rapport au diol.

13. Procédé selon la revendication 12, dans lequel le solvant à base de diol contient dans l'intervalle allant de 1,0 à 2,5 pour-cent en poids d'eau, calculés par rapport au diol.

14. Procédé selon la revendication 12 ou 13, dans lequel le solvant à base de diol contient moins de 700 parties par million en poids de ligand, calculées par rapport au solvant total.

15. Procédé selon l'une quelconque des revendications précédentes, dans lequel on utilise un rapport molaire du nickel au ligand d'au moins un.

16. Procédé selon l'une quelconque des revendications précédentes, dans lequel le ligand bidentate est un acide o-dihydrocarbylphosphinobenzoïque ou un de ses sels de métal alcalin.

17. Procédé selon la revendication 16, dans lequel le ligand bidentate est l'acide o-diphénylphosphinobenzoïque ou un de ses sels de métal d'alcalin.